# EUROPEAN PATENT SPECIFICATION

(11) **EP 0 783 892 B1**
(45) Date of publication and mention of the grant of the patent: **03.07.2002**
(21) Application number: 96308463.7
(22) Date of filing: 22.11.1996
(51) Int. Cl.: A61K 39/39

(54) **Method to increase and modify immune responses**
Verfahren zur Steigerung und Modifizierung von Immunantworten
Procédé pour augmenter et modifier des réponses immunitaires

(30) Priority: 18.12.1995 US 574352; 19.12.1995 US 575050
(43) Date of publication of application: 16.07.1997
(73) Proprietor: National Institute of Immunology, New Delhi 110067 (IN)
(72) Inventor: Rath, Satyajit, New Delhi 110067 (IN); Abraham, Roshini, New Delhi 110067 (IN); Singh, Nagendra, New Delhi 110067 (IN); Basu, Sandip K., New Delhi 110067 (IN); Bal, Vineeta, New Delhi 110067 (IN); Mukhopadhyay, Amitabha, New Delhi 110067 (IN)
(74) Representative: Ebner von Eschenbach, Jennifer

(56) References cited:
- THE JOURNAL OF IMMUNOLOGY, vol. 154, 1 January 1995, pages 1-8, XP002028274 ABRAHAM, R. ET AL.: "MODULATION OF IMMUNOGENICITY AND ANTIGENICITY OF PROTEINS BY MALEYLATION TO TARGET SCAVENGAR RECEPTORS ON MACROPHAGES"
- VACCINE, vol. 9, no. 5, 1 May 1991, pages 351-357, XP000215822 COOPER P.D. ET AL: "ALGAMMULIN, A NEW VACCINE ADJUVANT COMPRISING GAMMA INULIN PARTICLES CONTAINING ALUM: PREPARATION AND IN VITRO PROPERTIES"
- VACCINE, vol. 13, 1995, pages 1131-1137, XP000652640 LOFTHOUSE S.A. ET AL.: "HUMORAL AND CELLULAR RESPONSES INDUCED BY INTRADERMALLY ADMINISTERED CYTOKINE AND CONVENTIONAL ADJUVANTS"

## Description

### Field of the Invention

This invention provides a novel strategy for increasing the immunogenicity of antigens, as well as for modifying the type of immune responses generated. This invention has particular applicability to increasing and modifying the immunogenicity of vaccines.

### Background of the Invention

A major difficulty in generating effective immune responses to vaccines has been that, for the immune system, recognition of a target molecule [or 'antigen'] does not automatically mean that the entity associated with this antigen is really 'dangerous' to the body. Thus, responding to every antigen may be a waste of the body's resources and lead to undesired side effects. This difficulty is further complicated by the fact that the immune system utilizes different kinds of pathways of action for different kinds of infections. It must kill infected cells to prevent replication of viruses, and eliminate cancerous cells to stop their spread. The immune system must make antibodies with certain characteristics to deal with extracellular infections, and it must activate macrophages to kill the facultative parasites within them. Each situation thus needs a specific kind of response.
CASE: US 08/574352 ETC

Since target recognition by itself is not determinative as to the defensive steps required, the context in which the target is presented to the immune system becomes decisive in triggering the appropriate action upon target recognition. Antibody-making B cells and virus-infected cells-recognizing cytotoxic CD8 T cells are heavily dependent on CD4 T cells in order to generate a response, since the CD4 T cells are the main source of interleukin-2 [IL-2], which is a major proliferation factor in the immune system, and immune cell proliferation is a major prerequisite of a successful immune response. Thus, CD4 T cells are central to any immune response, and changes in the context in which a given antigen is recognized by them can change both the quantity and the quality of the resultant immune response (Pfeiffer et al. [1991] Immunol Rev. 123, 65 - 84 (1991)].

CD4 T cells recognize antigens as small, processed peptide fragments bound to MHC class II molecules (MHC is major histocompatibility complex) on the surfaces of the cells which process antigen, the so-called antigen-presenting cells [APCs]. MHC class II molecules normally present peptides generated from extracellular antigens [such as most vaccines] in the peripheral endosomal compartment of the APC. MHC class II molecules are restricted in their tissue distribution, and macrophages, dendritic cells and B cells are the major lineages amongst the few that do express these molecules. This limits the APC function for CD4 T cells mainly to these cell lineages, making them 'professional' APCs for CD4 T cells. The abilities of these professional APCs to take up antigens from the microenvironment are also quite different, as shown by the fact, for example, that macrophages are far more capable of taking up particulate antigens such as bacteria or alum particles by phagocytosis than the other professional APCs.

In addition to this 'first' signal from specific peptide-MHC complexes, T cells need other signals from APCs for complete activation, -the contextual, so-called 'co-stimulatory' signals which do not activate T cells by themselves but only when the first signal has already been provided. These accessory molecules are also expressed differentially on various APC types, making these cell lineages very distinct in terms of the quantity of co-stimulation they can provide [Weaver and Unanune [1990] Immunol Today 11, 49-55].

Thus the signal from the APC to the CD4 T cell is heavily dependent on the abilities of the APC to take up antigens, to process them, to choose peptides that can bind to their MHC class II molecules and to express reasonable levels of these; - all of which affect the delivery of the first signal, - as well as the kind and quantity of accessory molecules on these APCs, which would affect the delivery of the co-stimulatory signal.

In practical terms, if diphtheria toxoid [DT] is injected into animals in aqueous solution, there is hardly any immune response to it. However, if it is injected adsorbed on an adjuvant like alum as in a conventional anti-DT vaccine preparation, it evokes a significant response. One of the problems with alum as an adjuvant is its relatively low adjuvanticity in comparison with other adjuvants, such as Freund's adjuvants which are not licensed for use in humans. Another is its poor ability to activate an immune pathway of action that leads to the production of interferon-gamma [IFN-g]. IFN-g activates macrophages to kill parasites residing within them [Buchmeir et al. [1985] Proc Natl Acad Sci USA 82, 7404, 7408] and is therefore of great utility in many infections.

### Summary of the Invention

Most theories regarding the basis of adjuvanticity suggest that making antigens available to active APCs over long periods of time is the basis for the improved immune response. Hence, applicants have been attempting to deliver antigens to a specific APC. type and the present'invention is the result of these experiments.

Antigens can be modified to increase the immune response. An immune response can also be modified by adsorbing an antigen, and a compound that binds scavenger receptors onto an adjuvant. The present invention allows modification of adjuvants, such as alum, such that when adjuvanticity is enhanced, the ability to trigger an IFN-g-producing pathway is increased.

Therefore, an object of this invention is to target antigen to macrophage scavenger receptors as a means of enhancing the immunogenicity of such proteins. A further object is to enhance the immunogenic effects of known adjuvants like alum. It is possible to enhance the immunogenic effects by a direct chemical modification of the antigen by attaching acyl or carboxyl groups to the antigen (for example, by reacting the antigen with an acid anhydride e.g. by maleylation, succinylation, citraconylation or acetylation, or any combination thereof). However, the object of this invention is to enhance the immunogenic effects by co-adsorption of the antigen with another scavenger receptor ligand. Yet another object is to change the cytokine dominance in the triggered T cells towards IFN-g, in order to bring about more effective macrophage activation against known infectious diseases such as tuberculosis, leprosy, typhoid fever, as well as for purposes of redirecting antibody responses away from IgE in therapy of allergies.

Accordingly, the present invention provides the use of an antigen and a scavenger receptor ligand that are coadsorbed or chemically conjugated with an adjuvant for the preparation of a medicament for modifying an immune response in a mammal, wherein the scavenger receptor ligand is polyguanylic acid.

In a further aspect of the invention there is provided a vaccine which is characterized by an antigen and a scavenger receptor ligand that are coadsorbed or chemically conjugated with a pharmaceutically acceptable adjuvant wherein the scavenger receptor ligand is polyguanylic acid.

### Brief Description of the Drawings

Figure 1 illustrates T cell responses to immunizing antigen from mice immunized with DT-saline [hollow circles] or mDT-saline [solid circles].

Figure 2 illustrates various maleylated proteins showing serological cross-reactivity with each other but not with their native counterparts.

Figure 3 illustrates that T cell proliferative responses of mDT-immunized mice can be recalled efficiently by DT. The responses to both mDT [solid circles] and DT [solid triangles] are shown. The background ³H-thymidine incorporation by spleen cells alone was less than 2,000 cpm.

Figure 4 illustrates that the higher T cell proliferative response of mDT-immune cells to mDT [solid circles] can be blocked by competing mBSA [hollow circles], but the response to DT [solid triangles] is completely unaffected [hollow triangles]. The background ³H-thymidine incorporation by spleen cells alone was 4500 cpm.

Figure 5 illustrates serum anti-DT antibody levels in mice [groups of 4-6 mice each] cross-primed with various antigens in saline before a single dose of DT in saline.

Figure 6 illustrates serum anti-DT antibody levels in mice [groups of 4-6 mice each] cross-primed with various antigens on alum before a single dose of DT in saline.

Figure 7 illustrates serum anti-DT antibody levels over time in mice cross-primed with various antigens before a single dose of DT in saline.

Figure 8 illustrates proliferation responses in vitro to DT of T cells from DT-alum-immune or mDT-alum-immune mice.

Figure 9 illustrates IgGl/IgG2a ratios in serum anti-DT antibodies of mice already primed with various immunogen formulations before administration of DT in saline. Mean ± SE values of 4-6 mice are shown for each group.

Figure 10 illustrates IgG1/IgG2a ratios in serum anti-TT antibodies of mice already primed with various immunogen formulations before administration of TT in saline. Mean ± SE values of 4-6 mice are shown for each group.

Figure 11 illustrates cytokine levels produced by T cells from variously immunized mice as shown upon stimulation in vitro with 30 ug/ml mDT. IFN-g is shown as ng/ml and IL-4 as 10⁻¹ U/ml.

### Detailed Description of the Invention

Applicants' rationale for targeting antigens to macrophages is based on the fact that mature macrophages amongst cells capable of presenting antigens professionally have a unique surface receptor; -the scavenger receptor, -which recycles rapidly, works well against a steep concentration gradient, has no reported negative feedback control and delivers ligands into the endolysosomal pathway [Goldstein et al. [1979] Proc Natl Acad Sci USA 76, 333-337]. This makes it an ideal vehicle to deliver antigens to a specific APC lineage, namely, macrophages.

A variety of simple chemical modifications of antigens such as proteins can be made to confer a negative charge to the antigen that aids in its ability to bind to scavenger receptors. This will increase and/or modify the immune response. The modification is achieved by coupling one or more amino groups on the antigen with acyl and/or carboxyl groups. Attachment of acyl and/or carboxyl groups to antigens can be done by known reactions. Modification of antigens such as proteins is commonly accomplished by reacting the protein with appropriate acid anhydrides such as maleic, succinic, citraconic and acetic acid anhydrides. Other acid anhydrides can also be used. The antigen can be modified by maleylation, succinylation, citraconylation or acetylation. Methods to maleylate, succinylate, citraconylate and acetylate molecules are known in the art. A method of maleylation of proteins is described in Abraham et al., The Journal of Immunology, (1995, Vol. 154, pages 1-8). Maleylation can also be carried out by taking the protein in phosphate-buffered saline [pH 7.4] at a concentration of 5 mg/ml. The pH is then adjusted to 8.5 with 1 N sodium hydroxide. Previously weighed maleic anhydride [12.5 mg per ml of protein solution] is finely powdered and small amounts are added gradually to the protein solution with constant stirring and maintaining the pH between 8.5 and 9.0 by the addition of 1 N sodium hydroxide as necessary. After all the maleic anhydride has been added, the pH of the solution is allowed to drop to 7.4. The solution is then dialyzed against phosphate-buffered saline.

Maleylated or similarly modified antigens do not need to be associated with independent negatively charged molecules to increase or modify an immune response, since they themselves can now bind to the scavenger receptors. If they are adsorbed onto adjuvants such as alum particles, they will direct efficient uptake of the adjuvants by macrophages by virtue of binding to scavenger receptors.

Adjuvants are materials that, when given in association with antigens, increase the immune responses to these antigens.

A great variety of materials have been shown to have adjuvant activity. Adjuvants usually potentiate immune responses and may be specific or nonspecific in action [1]. These materials include protein carriers containing T cell epitopes, depot formers-which are water-oil or oil-water emulsions, polyclonal T cell activators like PPD, surface-active agents - saponin, Quil A, macrophage stimulators such as MDP and LPS and inert carriers like alum, bentonite or latex particles [2]. The most commonly used adjuvant for experimental work is Freund's Complete Adjuvant [FCA] which essentially contains killed mycobacteria emulsified in mineral oil. However, this preparation has not been approved for human use [3]. Among other adjuvants are immunostimulatory complexes [ISCOMs], where repetitive units of the antigen are held together by purified saponin [Quil A] and which have been shown to boost antibody levels [4]. Muramyl dipeptide [MDP] and its different derivatives are now being increasingly used for human vaccine studies [5]. MDP, a macrophage stimulator, has been reported to be the smallest unit capable of replacing whole mycobacteria in FCA [6]. The sodium phthalyl derivative of lipopolysaccharide [SP-LPS] has also been used in certain clinical studies with birth control vaccines [7]. This adjuvant can be used in the aqueous phase [8].

Preferred adjuvants for use in this invention are particulate adjuvants. A preferred particulate adjuvant is alum. A preferred adjuvant is colloidal alum, [Al(OH)₃] onto which the antigens to be immunized against are adsorbed. Alum, may be used in the insoluble particulate form as an adjuvant.

Alum bearing compounds are the only preparations allowed for general medical use in humans. Though alum is completely safe for medical use, it is not very effective in enhancing immune responses, especially cell-mediated responses. There have been many attempts to enhance the adjuvantic effects of alum - one approach has been to embed alum in the particles of an immune stimulant like gamma inulin, to form algammulin, in such a way that the alum is still capable of adsorbing the protein [9].

In accordance with the invention, unmodified antigens can be targeted to the scavenger receptors by binding them and the negatively charged molecule, polyguanylic acid, which has the ability to bind to scavenger receptors on macrophages, onto particle adjuvants thereby making the adjuvant target to macrophages carrying the antigens with it. Examples of other such negatively charged molecules are dextran sulfate, and fucoidin. Preferred adjuvants are particulate adjuvants. Alum is a preferred adjuvant. The combination of an antigen and a negatively charged molecule with adjuvants also improves the efficiency of adjuvants for eliciting effective immune responses.

Antigens, modified or unmodified, are adsorbed onto the adjuvant by mixing them together and incubating. Antigens and negatively charged compounds are adsorbed onto the adjuvant by mixing them together and incubating. For example, when alum is used as the adjuvant, a solution containing 1-10 mg/ml of the material to be adsorbed (singly or as a mixture) is made in a buffer at physiolgical pH [e.g. phosphate buffered saline, pH 7.4]. It is mixed with an equal volume of a commercial preparation of sterile alum particles [e.g. as supplied by Superfos Biosector, Denmark. According to the manufacturers, protein equivalent of up to 17 mg/ml can be adsorbed on the alum preparation at 1:1 (v/v) ratio] and incubated for 12-16 hours at 4°C. on a rocker at 10-40 r.p.m.

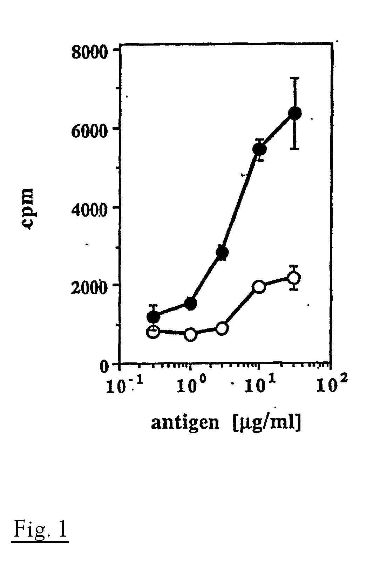

Applicants have demonstrated that both at the antibody level [table 1] and the T cell level [figure 1], maleylated proteins such as maleyl-DT [mDT] or maleyl-bovine serum albumin [mBSA] are more immunogenic than their non-maleylated counterparts when injected without adjuvant [Abraham et al. [1995] Immunol 154, 1-8].

Table 1 shows serum antibody responses in mice immunized with native or maleylated antigen in saline.

| Immunogen | Serum anti-immunogen antibody concentration [ng/ml] Mean ± S.E. [n=5] |
|---|---|
| DT | <30 |
| mDT | 215±40 |
| | |
| BSA | <30 |
| mBSA | 2500±400 |

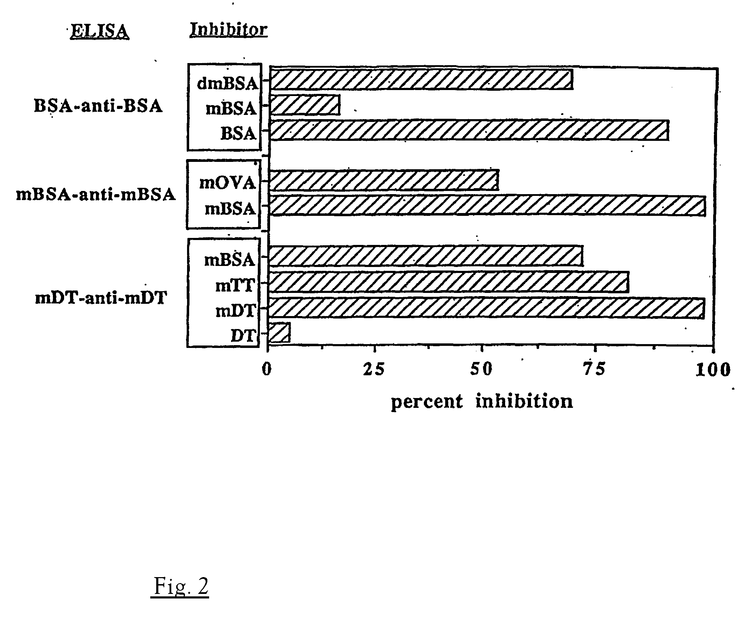

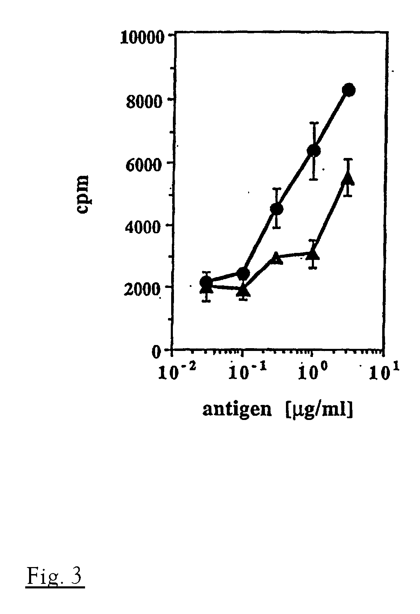

B cell epitopes of native and maleylated proteins are non-identical [Figure 2], but the T cell epitopes are similar [Figure 3] [Abraham et al. [1995] J Immunol 154, 1-8].

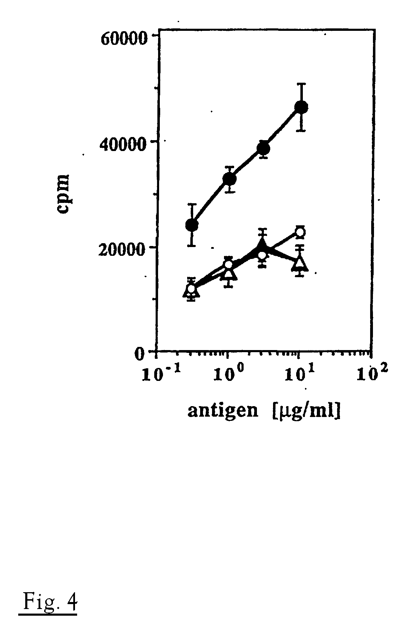

When applicants measured T cell response from such immunized mice in vitro, they found that the maleylated protein is much better presented to T cells than its native counterpart. An unrelated scavenger receptor ligand, maleyl-bovine serum albumin [mBSA], blocks the high level of presentation of maleyl-diphtheria toxoid [mDT], but does not enhance the presentation of native DT at all, establishing that enhanced uptake of the maleylated protein, rather than signaling by engagement of the scavenger receptors, is responsible for increased presentation [Figure 4] [Abraham et al. [1995] Immunol 154, 1-8].

These data together prove that the enhanced immunogenicity consequent upon targeting of proteins to scavenger receptors on macrophages is restricted to the scavenger receptor ligand and does not result from any non-specific increase in the ability of macrophages to present antigen.

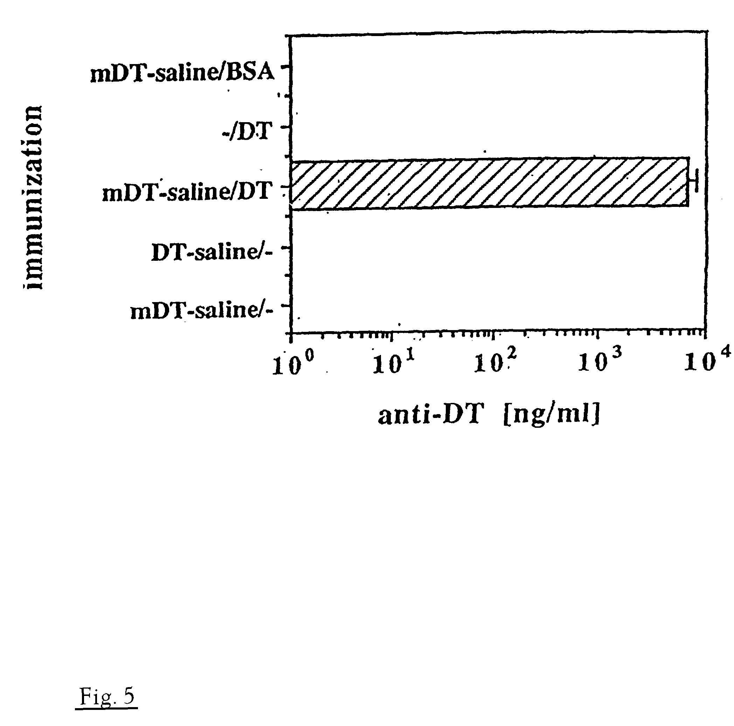

Maleylation of protein antigens provides a tool to prime T cells against a given protein without simultaneously generating antibodies against it. This allows 'cross-priming' experiments where the first immunization, or 'priming', is against maleylated antigen, followed by the administration of native antigen, followed by the administration of native antigen in solution without adjuvant. Priming with DT without adjuvant does not generate any antibodies, but a single dose of DT in saline after priming by mDT in saline generates significant anti-DT levels, though BSA does not generate any antibodies, showing that the effect is antigen-specific [Figure 5].

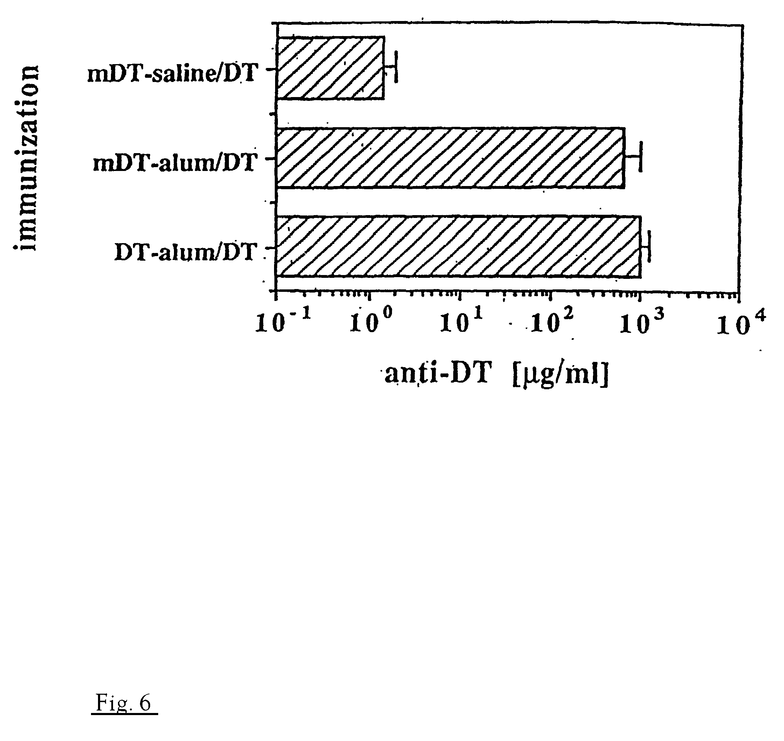

The anti-DT response generated by priming with mDT-alum is greater than that triggered by mDT in saline. Interestingly, the anti-DT response elicited by a single dose of DT in saline after mDT-alum priming is as good as the anti-DT response generated by DT-alum [Figure 6].

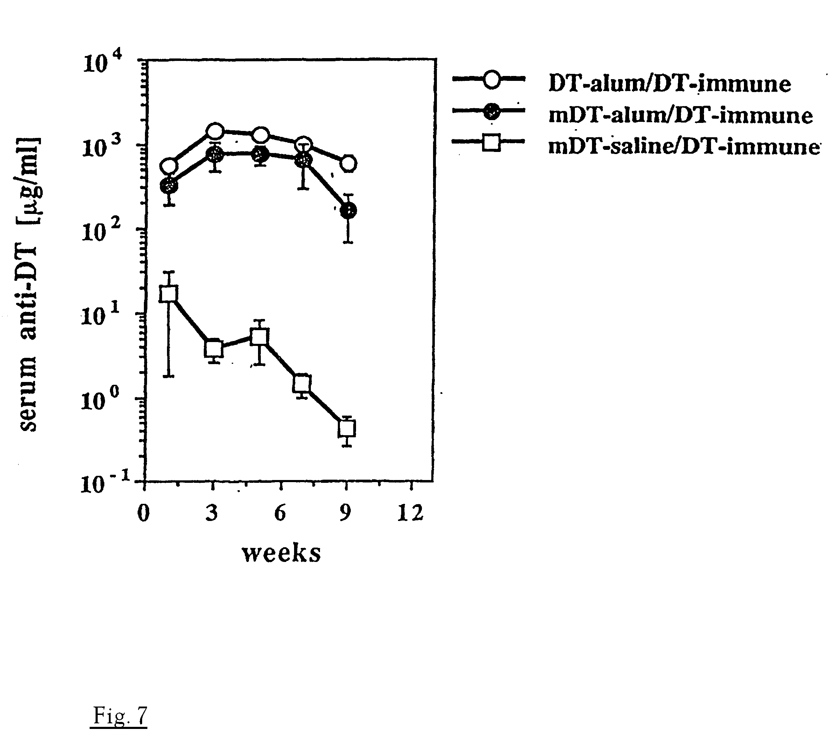

In fact, if these responses are followed over a period of time, the anti-DT antibody response triggered by DT in saline after mDT-alum and that elicited by DT-alum itself stay comparable, while the anti-DT triggered by mDT-saline declines [Figure 7].

These data make two points. One, B cell responses can be generated and maintained by soluble antigens if T cells are pre-primed, which suggests that adjuvanticity is primarily a T cell requirement. Two, maleylation and alum adsorption seem to have an additive effect for T cell adjuvanticity.

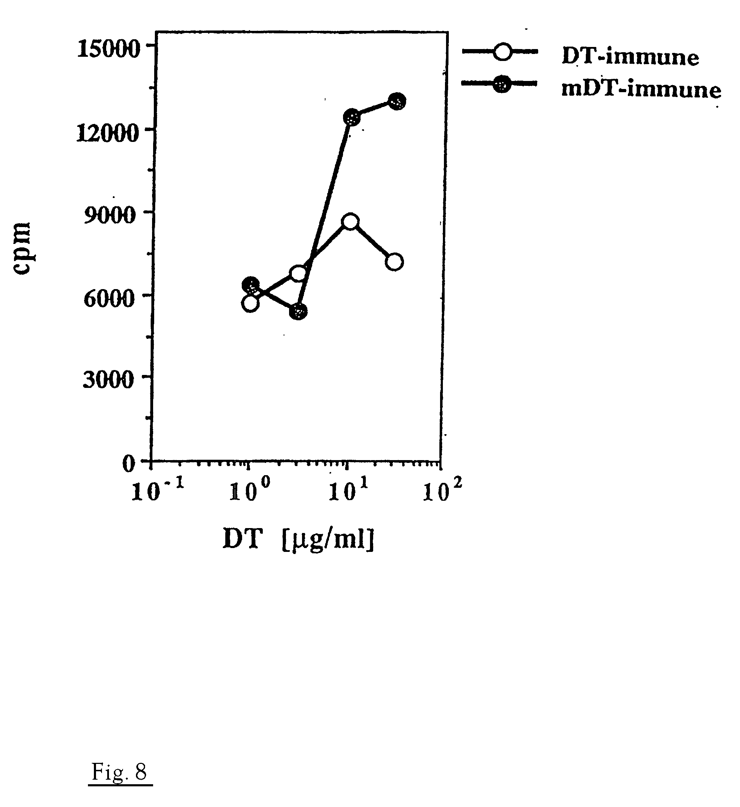

Applicants have established this latter possibility of an additive effect for T cell adjuvanticity between alum and maleylation by doing direct T cell proliferation assays from immunized mice, in which the response of mDT-alum-immune mice to native DT is clearly better than that of DT-alum-immune mice [Figure 8], showing that maleylation is a useful general strategy for enhancing the adjuvanticity of alum.

Until this point, the quantitative effects of the adjuvant properties added by protein maleylation to alum as an adjuvant have been described.

Applicants have also examined the effects of such maleylation in modulating the effector cytokines produced by the triggered T cells. CD4 T cells are known to commit themselves to one of two possible action pathways by making distinct 'packages' of cytokines which have very different biological effects. A 'Th1' response would thus make IFN-g and tumor necrosis factor-beta [TNF-b], but no interleukin-4[IL-4], IL-5 or IL-10, while a 'Th2' response would make the latter cytokines but not the former [Mossman et al. [1986] J Immunol 136, 2348]. The switching of antibody responses to IgG1 is interleukin-4-dependent [Snapper and Paul [1987] J Immunol 139, 10] and may be said to represent a 'Th2' or type 2 T cell response, while switching to IgG2a is IFN-g-dependent [Snapper et al. [1988] J Immunol 140, 2121] and thus would be a 'Th1' or type 1 response. The ratio of the two isotypes in an antibody response is thus a measure of the relative prominence of type 1 versus type 2 T cell responses.

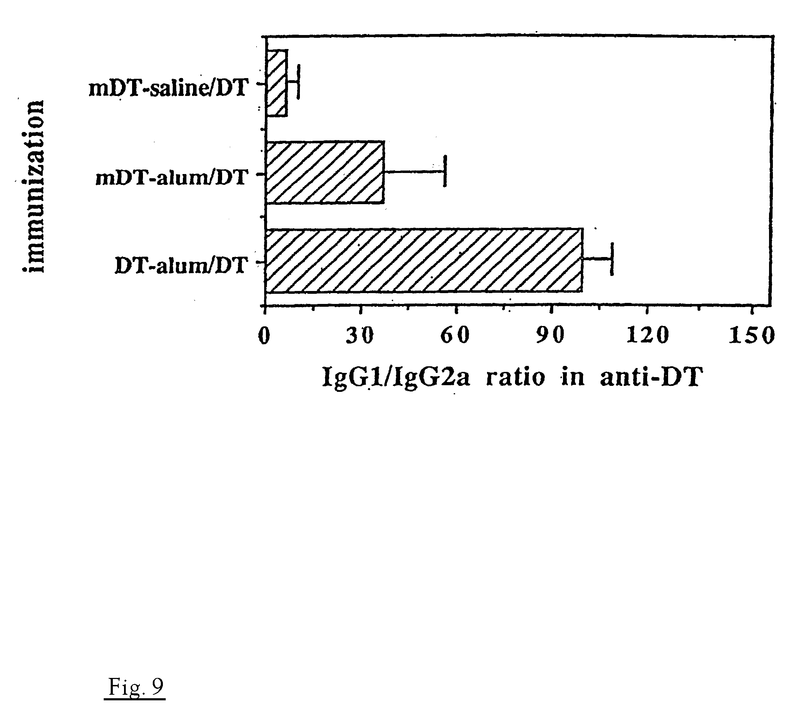

Applicants have measured the IgG1/IgG2a ratios in the cross-priming experiments described above. When the IgGl/IgG2a ratios in the anti-DT antibodies generated by DT-alum are compared with those generated by mDT-alum, the latter show a significantly greater prominence of IgG2a and therefore of IFN-g, and this is further enhanced in the mDT-saline-primed group [Figure 9].

The IgG1/IgG2a ratio in antibodies generated by non-targeted, native DT in saline cannot be measured, since DT in saline is not able to generate antibodies by itself in the protocol used here.

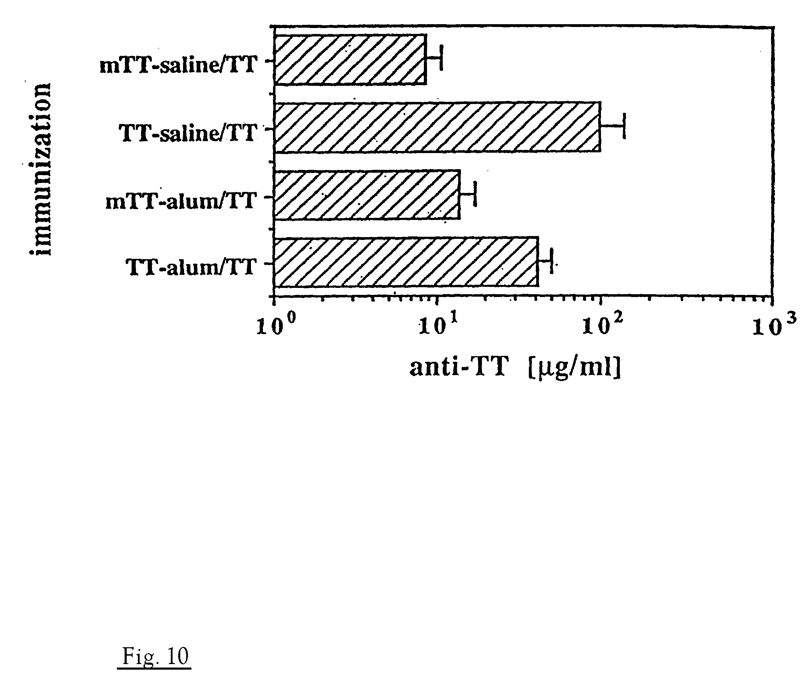

However, tetanus toxoid [TT] is immunogenic even in saline in the protocol of immunization used, and therefore the use of TT in an experiment similar to the above addresses this issue as well as provide support for the general applicability of the adjuvant effect of maleylation and its additive nature with alum. Mice in this experiment have been given TT or mTT, either in saline or on alum, followed by TT in saline for all groups as in the cross-priming experiment described above for DT. The IgG1/IgG2a ratios show very clearly that soluble non-targeted TT generates the least relative prominence of IFN-g, as compared to immunization with a particulate TT preparation on alum. This prominence of IFN-g increases significantly further if the alum particles are coated with maleylated antigen leading to improved receptor-mediated phagocytosis by macrophages, and it increase still further if one cuts down on the non-specificity in the targeting by decreasing the half-life of the targeting formulation by giving it as a rapidly clearing, targeted antigen in saline [Figure 10].

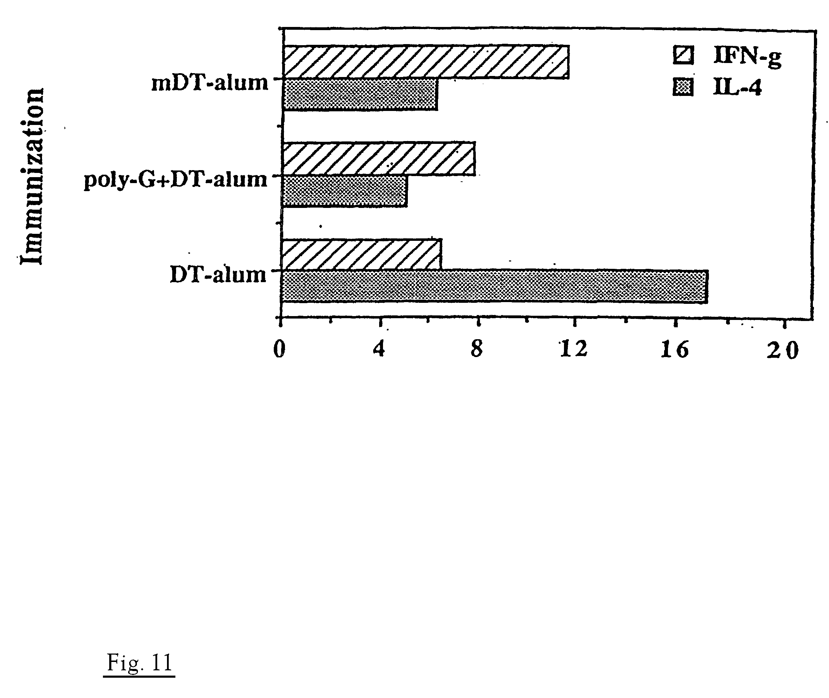

Applicants have also examined the ability of non-proteinaceous ligand of scavenger receptors, polyguanylic acid [poly-G] [Brown et al. [1980] J Supramol Struct 13, 67], to modulate the cytokine profiles of the triggered T cells when co-adsorbed with DT on alum so as to induce uptake of these alum particles by macrophages with greater efficiency. For this, applicants have carried out direct cytokine analysis in culture supernatants of T cells from variously immunized mice upon stimulation with the appropriate antigens, and have shown that the relative quantities of IL-4 and IFN-g are predictably modulated with DT-alum-immune mice showing the highest relative level of IL-4, while Poly-G+DT-immune mice and mDT-immune mice show a relatively higher proportion of IFN-g [Figure 11].

Thus, macrophage targeting not only enhances immunogenicity, but also changes the cytokine profile of the triggered T cells to skew it towards IFN-g, and alum and scavenger receptor targeting show an additive effect for both these properties.

### References

1. Allison, A.C. and Byars, N.E. An adjuvant formulation that selectively elicits the formation of antibodies of protective isotypes and of cell-mediated immunity. J. Immunol. Methods 1986. 95: 157.
2. Allison, A.C. and Byars, N.E. Immunological adjuvants; desirable properties and side effects. Mol. Immunol. 1991. 28: 279.
3. Claassen, E. and W.J.A. Boersma. Characteristics and practical use of new generation adjuvants as an acceptable alternative to Freund's complete adjuvant. 1992. Res. Immunol. 143: 175.
4. Jones, P.D., Tha Hla, R., Morein, B. et al. Cellular immune responses in the murine lung to local immunization with influenza A virus glycoproteins in micelles and ISCOMs. 1988. Scand. J. Immunol. 27: 645.
5. Jones, W.R. Bradley, J., Judd, S.J. et al. Phase I clinical trial of a World Health Organization birth control vaccine. 1988. Lancet 1: 1295.
6. Ellouz, F., Adam, A., Ciorbaru, R. and Lederer, E. Minimal structural requirements for adjuvant activity of bacterial peptidoglycan derivatives. 1974. Biochem. Biophys. Res. Commun. 59: 1317.
7. Talwar, G.P., Singh, O., Pal, R., Chatterjee, N., Sahai, P., Dhall, K., Kaur, J., Das, S.K., Suri, S., Buckshee, K., Saraya, K. and Saxena, B.N. A vaccine that prevents pregnancy in women. 1994. Proc Natl. Acad. Sci. U.S.A. 91: 8532.
8. Elin, R.J., Wolff, S.M., McAdam KPWJ et al. Properties of Escherichia coli endotoxin and its phthalylated derivative in humans. 1981. J. Inf. Dis. 144: 329, page 16.
9. Cooper, P.D. and Steele, E.J. Algammulin, a new vaccine adjuvant comprising gamma inulin particles containing alum: preparation and in vivo properties. 1991. Vaccine. 9: 351.

## Claims

1. Use of an antigen and a scavenger receptor ligand that are coadsorbed or chemically conjugated with an adjuvant for the preparation of a medicament for modifying an immune response in a mammal wherein the scavenger receptor ligand is polyguanylic acid.

2. Use according to claim 1 wherein the adjuvant is alum.

3. A vaccine **characterized by** an antigen and a scavenger receptor ligand that are coadsorbed or chemically conjugated with a pharmaceutically acceptable adjuvant wherein the scavenger receptor ligand is polyguanylic acid.

4. A vaccine according to claim 3 wherein the adjuvant is alum.

## Patentansprüche

1. Verwendung eines Antigens und eines Scavenger-Rezeptor-Liganden, die coadsorbiert oder chemisch konjugiert sind mit einem Adjuvans, für die Herstellung eines Medikaments zum Modifizieren einer Immunantwort in einem Vertreter der Mammalia, wobei der Scavenger-Rezeptor-Ligand Polyguanylsäure ist.

2. Verwendung nach Anspruch 1, wobei das Adjuvans Alaun ist.

3. Vakzine, **gekennzeichnet durch** ein Antigen und einen Scavenger-Rezeptor-Liganden, die coadsorbiert oder chemisch konjugiert sind mit einem pharmazeutisch zulässigen Adjuvans, wobei der Scavenger-Rezeptor-Ligand Polyguanylsäure ist.

4. Vakzine nach Anspruch 3, wobei das Adjuvans Alaun ist.

## Revendications

1. Utilisation d'un antigène et d'un ligand de récepteurs *scavenger* qui sont co-adsorbés ou chimiquement conjugués avec un adjuvant pour la préparation d'un médicament destiné à modifier une réponse immunitaire chez un mammifère, dans laquelle le ligand du récepteur *scavenger* est l'acide polyguanylique.

2. Utilisation selon la revendication 1, dans laquelle l'adjuvant est l'alun.

3. Vaccin **caractérisé par** un antigène et un ligand de récepteurs *scavenger* qui sont co-adsorbés ou chimiquement conjugués avec un adjuvant acceptable du point de vue pharmaceutique, dans lequel le ligand du récepteur *scavenger* est l'acide polyguanylique.

4. Vaccin selon la revendication 3, dans lequel l'adjuvant est l'alun.
